# EUROPEAN PATENT APPLICATION

(11) **EP 4 524 880 A1**
(43) Date of publication of application: **19.03.2025**
(21) Application number: 23196929.6
(22) Date of filing: 12.09.2023
(51) Int. Cl.: G06T 7/00, G06T 7/11, G16H 30/40, G16H 50/20

(54) **DETECTING REGION OF INTEREST IN 3D IMAGE DATA OF PROSTATE**

(71) Applicant: Koninklijke Philips N.V., 5656 AG Eindhoven (NL)
(72) Inventor: MYSORE SIDDU, Dinesh, Eindhoven (NL); ADAK, Saptakatha, Eindhoven (NL); PAWAR, Anil, 5656AG Eindhoven (NL); FONOLLA NAVARRO, Roger, Eindhoven (NL); GAYET, Maudy, Eindhoven (NL); HOFFMANN, Ralf Dieter, Eindhoven (NL)
(74) Representative: Philips Intellectual Property & Standards

(57) **Abstract**

A detection system and method are provided for detecting a region of interest in three-dimensional [3D] image data of a prostate of a patient. For that purpose, the 3D image data is segmented into at least two glandular regions, and for each of the glandular regions, a separate machine learning model is provided, which is trained to detect a region of interest in the 3D image data of a respective glandular region and during inference applied to the 3D image data of the respective glandular region to obtain a detection result. By using separate machine learning models for each of the glandular regions, the models may be specialized for the specific characteristics of a region of interest in each glandular region. To train the machine learning models, a training system and method are provided.

## Description

### FIELD OF THE INVENTION

The invention further relates to a computer-implemented method and detection system for detecting a region of interest in three-dimensional [3D] image data of a prostate. The invention further relates to a computer-implemented method and training system for training a plurality of machine learning models to detect a region of interest in 3D image data of a prostate. The invention further relates to a computer-readable medium comprising instructions to cause a processor system to perform any of the methods.

### BACKGROUND OF THE INVENTION

The prostate gland is a critical component of the male reproductive system, and its health is essential for the overall well-being of an individual. Various ailments can affect the prostate, with prostate cancer being one of the most severe and common. Accurate and early detection of regions of interest (ROI) within the prostate that are suspicious for cancerous lesions is imperative for timely intervention and treatment. In this context, medical imaging is an invaluable tool for non-invasive examination of the prostate.

Conventionally, radiologists have relied on magnetic resonance imaging (MRI) for visualizing the internal structure of the prostate. Among various MRI techniques, T2-weighted (T2W) images have been particularly favoured for detecting regions suspicious for prostate cancer. The T2W images offer enhanced contrast, which makes it easier to discern between normal and abnormal tissues. Radiologists usually perform the delineation of the region of interest by contouring the prostate in a slice-by-slice manner using different views - axial, sagittal, or coronal, or a combination of these views. This meticulous manual detection process, although effective, is labour-intensive and time-consuming.

In recent times, healthcare systems across the globe have been facing acute shortages in medical staff, which exacerbates the burden on radiologists. Moreover, the manual detection process is subject to human error and variations in interpretation among radiologists. As such, it is highly desirable to develop automated systems that can accurately segment regions of interest within the prostate. Automated detection not only has the potential to relieve the workload on medical staff but also serves as an invaluable second opinion, which can enhance the reliability of the diagnostic process.

While automated segmentation systems have been developed, see for example [1], segmenting the prostate gland presents unique challenges. The prostate is a complex organ, with substantial variations in size, shape, and appearance among individuals. Additionally, the prostate's morphology can change due to various factors, including age, disease conditions, and other physiological variables. These factors contribute to a high degree of variability in the size and shape of the prostate gland, which in turn leads to variability in the segmentation of regions of interest in the prostate gland.

Achieving accurate and reliable segmentation of regions of interest in the prostate is a challenging task. It is therefore desirable for be able to detect regions of interests in the prostate while handling the varied nature of the prostate gland and produce results with a low number of false positives and negatives, so as to ensure that the patients receive the most accurate diagnosis and, consequently, the most effective treatment plan.

### References

[1] Rouvière O, et al. Combined model-based and deep learning-based automated 3D zonal segmentation of the prostate on T2-weighted MR images: clinical evaluation. Eur Radiol. 2022 May.

### SUMMARY OF THE INVENTION

In a first aspect of the invention, a computer-implemented method is provided for detecting a region of interest in three-dimensional [3D] image data of a prostate of a patient, comprising:
accessing the 3D image data;
segmenting the 3D image data into at least two glandular regions;
for each of the glandular regions:
   - providing a separate machine learning model which is trained to detect a region of interest in the 3D image data of a respective glandular region,
   - applying the separate machine learning model to the 3D image data of the respective glandular region to obtain a detection result,
thereby obtaining a plurality of detection results for the glandular regions;
outputting detection data indicative of the plurality of detection results.

In a further aspect of the invention, a detection system is provided for detecting a region of interest in 3D image data of a prostate of a patient, comprising:
an input interface for accessing the 3D image data;
a processor subsystem configured to:
   - segment the 3D image data into at least two glandular regions;
   - for each of the glandular regions:
      - provide a separate machine learning model which is trained to detect a region of interest in the 3D image data of a respective glandular region,
      - apply the separate machine learning model to the 3D image data of the respective glandular region to obtain a detection result,
thereby obtaining a plurality of detection results for the glandular regions;
an output interface for outputting detection data indicative of the plurality of detection results.

In a further aspect of the invention, a computer-implemented method is provided for training a plurality of machine learning models to detect a region of interest in 3D image data of a prostate of a patient, comprising:
accessing training data comprising a plurality of training data instances, wherein a training data instance comprises 3D image data of a prostate of a patient and annotation data which provides an annotation of one or more regions of interest in the 3D image data;
providing a plurality of machine learning models, wherein a separate machine learning model is provided for each of at least two glandular regions of the prostate;
for each or at least a subset of the training data instances:
   - segmenting the 3D image data into the at least two glandular regions,
   - determining which of the at least two glandular regions comprises a region of interest for which an annotation is provided in the annotation data, and
   - based on said determined glandular region, selecting and training a corresponding machine learning model on the 3D image data using the annotation of the region of interest as prediction target,
thereby obtaining a plurality of trained machine learning models;
outputting model data defining the plurality of trained machine learning models.

In a further aspect of the invention, a training system is provided for training a plurality of machine learning models to detect a region of interest in 3D image data of a prostate of a patient, comprising:
an input interface for accessing training data comprising a plurality of training data instances, wherein a training data instance comprises 3D image data of a prostate of a patient and annotation data which provides an annotation of one or more regions of interest in the 3D image data;
a processor subsystem configured to, for each or at least a subset of the training data instances:
   - provide a plurality of machine learning models, wherein a separate machine learning model is provided for each of at least two glandular regions of the prostate;
   - for each or at least a subset of the training data instances:
segment the 3D image data into the at least two glandular regions,
determine which of the at least two glandular regions comprises a region of interest for which an annotation is provided in the annotation data, and
based on said determined glandular region, select and train a corresponding machine learning model on the 3D image data using the annotation of the region of interest as prediction target,
thereby obtaining a plurality of trained machine learning models;
an output interface for outputting model data defining the plurality of trained machine learning models.

In a further aspect of the invention, a transitory or non-transitory computer-readable medium is provided, the computer-readable medium comprising data representing a computer program comprising instructions for causing a processor system to perform a computer-implemented method as described in this specification.

The above measures involve training a plurality of machine learning models to be able to detect a region of interest in 3D image data of a prostate of a patient, and the subsequent use (also referred to as 'inference' in the field of machine learning) of the plurality of machine learning model for the aforementioned detection. This use may for example take place during screening or treatment of a patient.

The training of the machine learning models may involve providing training data which comprises a number of training data instances. Each or at least a subset of these training data instances may comprise 3D image data of a prostate of a patient, for example 3D image data acquired by an MRI system. In addition, annotation data may be provided, which annotation data may provide an annotation of a region of interest in the 3D image data, and if there are multiple regions of interest in the 3D image data, multiple of such annotations. The region of interest may for example be a region which is suspicious for a cancerous lesion or a region which is deemed atypical for other reasons. An annotation may take various forms, such as a segmentation of a region of interest through a delineated outline, or a mask that highlights a specific region or masks out its background. The annotation data may provide any number of such annotations, for example zero (e.g., in case there are no regions of interest in the 3D image data), one, or a plurality of annotations. Such annotations may typically be obtained by manual annotation, for example by a radiologist or other clinical staff. The training data instances may typically relate to different patients. In some examples, multiple training data instances may relate to the same patient, but may differ in terms of 3D image data, for example by having been acquired at different time instances, and/or in annotation data, e.g., by comprising annotations provided by different radiologists.

The training may further involve segmenting the 3D image data of a particular training instance into at least two glandular regions. Such glandular regions may for example include the peripheral region (also called `peripheral zone') of the prostrate and a central gland region of the prostrate, with the latter comprising the anterior fibromuscular stroma, the transition zone, and the central zone of the prostate. However, this is not a limitation, in that any other glandular regions may be segmented. Each glandular region may be a 3D region and its segmentation may result in for example a 3D contour or 3D mask. The glandular regions are typically non-overlapping and may together cover at least a part, or in some cases even all of the prostate gland.

The training may further involve providing a separate machine learning model for each of the aforementioned glandular regions. Here, the term 'separate' may refer to the respective models being different instances of a same type of model, e.g., a deep neural network, but with each instance having different trainable model parameters, or the models being of the same type but having a different configuration, e.g., a different number of layers, a different size of the layers, etc, or the models being of a different type, e.g., a deep neural network model and a random forest model.

Having segmented the 3D image data of a particular training data instance, it may be determined which of the at least two glandular regions comprise a region of interest for which an annotation is provided in the annotation data. This may for example involve comparing coordinates of an annotation to coordinates of the segmentation of a glandular region. If one of the glandular regions is determined to comprise a region of interest, the corresponding machine learning model may be trained, e.g., in a training iteration, on the 3D image data of the particular glandular region and using the annotation of the region of interest as a prediction target. This way, the machine learning model may be trained to detect regions of interest in the image data of this particular glandular region. In some examples, if different glandular regions each comprise a region of interest, the corresponding machine learning models may be trained on the respective 3D image data using the respective annotations as prediction targets. In some examples, if there is no region of interest specified in a particular glandular region, for example by the region of interest lying in another glandular region or there not being any region of interest defined by the annotation data, the corresponding machine learning model may still be trained on the 3D image data of the particular glandular region but using a prediction target which represents an absence of a region of interest. In other words, the machine learning model may be trained to refrain from detecting a region of interest in the 3D image data of a particular glandular region.

Having trained the plurality of machine learning models on the training data, the trained machine learning models may be deployed in clinical practice. During use ('inference'), 3D image data of a new patient may be segmented into the at least two glandular regions and each machine learning model may be applied to the image data of the corresponding glandular region so as to obtain a detection result. In other words, if there are two glandular regions for which a trained machine learning model is available, both machine learning models may be applied to the 3D image data of the respective glandular region to produce a detection result. The detection result may take the same or similar form as the annotation data used during training, being for example a contour or a mask. The detection results may then be combined to provide an identification of possible regions of interest for all of the aforementioned glandular regions. It will be appreciated that such a detection result may identify one or more regions of interest but may also indicate that the 3D image data does not contain a region of interest.

The above measures have the effect of improving the detection of regions of interest in 3D image data of a prostate. This may be explained as follows. The glandular regions have different characteristics. For example, different glandular regions may comprise different types of tissues which may appear differently in the 3D image data. For example, the peripheral zone is typically represented in the 3D image data a thin layer of homogeneous high signal intensity with a hypointense, well-defined, and non-interrupted capsula. The transition zone on the other hand typically shows heterogeneity of intermediate signal intensities focally replaced by well-circumscribed hyperplastic nodules of benign prostatic hyperplasia. As such, regions of interest may have different characteristics in each of the glandular regions. By using separate models for each of the glandular regions, these models may be specialized for the specific characteristics of a region of interest in each glandular region. This specialization results in higher accuracy and performance for each specific detection task, as also demonstrated elsewhere in this specification. Namely, a single model trying to learn the features of multiple glandular regions may become overly complex, as the model has to understand a wide range of features and patterns, and may not perform optimally for all glandular regions as the model's capacity may get diluted in trying to generalize across varied data. By only having to learn the features of regions of interest in a specific glandular region, individual models may be less complex compared to a single, all-encompassing model and provide better performance as the individual models may only need to generalize across one glandular region. It may also be easier to fine-tune or modify a model dedicated to a single glandular region as one does not have to worry about the impact of such fine-tuning on other glandular regions. This allows for optimization focused on the specific challenges of each glandular region. Furthermore, with separate models for each glandular region, performance trade-offs, for example between sensitivity and specificity, may be optimized for each specific glandular region based on its importance and requirements. The use of separate models may also ensure that data imbalances across different glandular regions do not affect each other. Interestingly, while one may expect that when trying to segment different organs, the use of different segmentation techniques may be beneficial, it was found that also the detection of lesions in such regions may benefit from a differentiation between the regions. Most surprisingly, the prostate was found as an extraordinary case where such differentiation within a single organ is very beneficial.

The following embodiments may, unless otherwise noted, relate to each of the training system and corresponding computer-implemented method and the detection system and corresponding computer-implemented method, mutatis mutandis.

In an embodiment, the at least two glandular regions include a peripheral region of the prostrate and a central gland region of the prostrate. The characteristics of regions of interest in the peripheral region and the central gland region have been found to differ significantly. By providing separate models for each of these glandular regions, the accuracy and performance of the detection in each region may be improved.

In an embodiment, the central gland region comprises an anterior fibromuscular stroma, a transition zone, and a central zone of the prostate.

In an embodiment, each of the at least two glandular regions is segmented into at least two subregions which divide a respective glandular region along a cranio-caudal axis of the prostrate, wherein a separate machine learning model is applied to the image data of each of the at least two subregions. It has been found that there is also significant variability in the characteristics of regions of interest within each glandular region. In particular, the characteristics of regions of interest have been found to differ as a function of the position along the cranio-caudal axis of the prostate. By subdividing the glandular regions in at least two subregions along the cranio-caudal axis and applying a separate machine learning model to each of these subregions, the accuracy and performance of the detection in each (sub)region may be improved. For example, each glandular region may be divided such that it comprises a base subregion at a base of the prostate and an apex subregion at an apex of the prostrate.

In an embodiment, each of the at least two glandular regions is segmented into at least three subregions, wherein the at least three subregions comprise the base subregion, the apex subregion, and a middle subregion positioned between the base subregion and the apex subregion along the cranio-caudal axis. The division of the glandular regions, and in particular the division of the peripheral region and the central gland region of the prostrate, into at least three subregions has been found to further improve the accuracy and performance of the detection of regions of interest compared to the division into two subregions, or no subregions at all. Accordingly, at least six machine learning models may be provided and trained to detect regions of interest in each respective subregion, namely the base peripheral region, the middle peripheral region, the apex peripheral region, the base central gland region, the middle central base region, and the apex central gland region.

In an embodiment, the segmentation into glandular regions is performed using a machine learning model and wherein the segmentation into subregions is performed using heuristics or based on user-input. The inter-patient variability in the shape and size of glandular regions may be significant. As such, it may be beneficial to use a segmentation technique which is able to cope with such inter-patient variability, for example a machine learning model which is trained to perform the segmentation. Having obtained the segmentation of a particular glandular region, the subregions may be more easily obtained, for example by dividing the volume of the glandular region into two or three parts along the cranio-caudal axis. As such, such division into subregions, which may itself be considered a segmentation, may be performed using simpler heuristics or even manually. For example, the division may simply use the relative position along the cranio-caudal axis to determine where to divide the glandular region into subregions.

In an embodiment, applying the separate machine learning model to the 3D image data of the respective glandular region comprises masking-out or cropping-away image data outside of the respective glandular region. By masking-out or cropping-away the image data outside of a glandular region, a machine learning model may be trained and subsequently used to only detect regions of interest in the image data of a particular glandular region.

In an embodiment, the 3D image data is obtained by magnetic resonance imaging, for example using a T2 sequence.

In an embodiment, a respective machine learning model is a neural network comprising one or more convolutional layers. In particular, a respective machine learning model may be a deep neural network with a number of convolutional layers.

In an embodiment of the detection system, the detection system further comprises a display output interface for visualizing the plurality of detection results on a display, for example as an overlay over the 3D image data. This way, the detection system may visualize the detection results on a display. In some examples, the detection system may be a subsystem of a larger system, e.g., a medical image viewing system.

It will be appreciated by those skilled in the art that two or more of the above-mentioned embodiments, implementations, and/or optional aspects of the invention may be combined in any way deemed useful.

Modifications and variations of any system, any computer-implemented method and/or the computer program product, which correspond to the described modifications and variations of another one of said entities, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which:
Fig. 1 shows a detection system configured to detect a region of interest in 3D image data of a prostate of a patient using a plurality of machine learning models;
Fig. 2 shows a training system configured to train a plurality of machine learning models to detect a region of interest in 3D image data of a prostate of a patient;
Fig. 3A shows a cross-sectional view of a prostate gland;
Fig. 3B shows a sagittal cross-section of the prostate gland;
Fig. 4 shows an architecture of a nnU-Net machine learning model for segmenting glandular regions in 3D image data of a prostate;
Fig. 5A shows a segmentation of a prostate in an axial image slice;
Fig. 5B shows a segmentation of two glandular regions in the prostate, being a central gland region and a peripheral region;
Fig. 6A shows another example of the segmentation of glandular regions;
Fig. 6B illustrate a segmentation of a glandular region into subregions;
Figs. 7 and 8 each show a comparison between the detection of regions of interest by a machine learning model which is trained to detect regions of interest in the entire prostate region and by a plurality of machine learning models which are specifically trained to each detect regions of interest in a specific glandular region;
Fig. 9 shows a method for detecting a region of interest in 3D image data of a prostate of a patient using a plurality of machine learning models;
Fig. 10 shows a method for training the plurality of machine learning models;
Fig. 11 shows a non-transitory computer-readable medium comprising data.

It should be noted that the figures are purely diagrammatic and not drawn to scale. In the figures, elements which correspond to elements already described may have the same reference numerals.

### DETAILED DESCRIPTION OF EMBODIMENTS

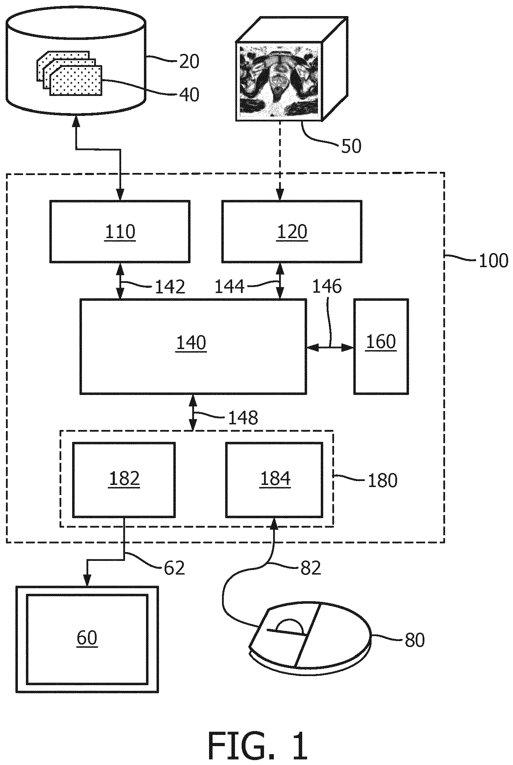

Fig. 1 shows a detection system 100 which is configured for detecting a region of interest in three-dimensional [3D] image data of a prostate of a patient. The detection system 100 is shown to comprise an input interface 120 to access the 3D image data 50. For example, the 3D image data 50 may be accessed via the input interface 120 from a Picture Archiving and Communication System (PACS) or an Electronic Medical Record (EMR) database of a Hospital Information System (HIS) to which the system 100 may be connected or comprised in. The detection system 100 may further comprise a data storage interface 110 to a data storage 20. The data storage 20 may serve as short term and/or long-term data storage. For example, the 3D image data 50 obtained via the input interface 120 may be at least temporarily stored on the data storage 20. In some embodiments, the 3D image data 50 may also be accessed from the data storage 20 instead of using another input interface. In the example of Fig. 1, the data storage interface 110 is shown to be connected to an external data storage 20. Alternatively, the data storage 20 may be an internal data storage of the detection system 100 (not shown in Fig. 1). In general, the data storage interface 110 as well as the input interface 120 may take various forms, such as a hard disk or solid-state disk interface to one or more hard disks and/or solid state disks, or a network interface to a Local Area Network (LAN). In some embodiments, the input interface 120 and the data storage interface 110 may be a same interface.

The detection system 100 may further comprise a processor subsystem 140 configured to internally communicate with the input interface 120 via data communication 144, with the data storage interface 110 via data communication 142, with a memory 160 via data communication 146 and with a user interface subsystem 180 via data communication 148. The memory 160 may for example be a volatile memory in which a computer program may be loaded which may cause the processor subsystem 140 to carry out functions which are described in this specification as being performed by the detection system 100.

The user interface subsystem 180 may be an optional component of the detection system 100, and if present, may be configured to, during operation of the detection system 100, enable a user to interact with the detection system 100, for example using a graphical user interface. The user interface subsystem 180 is shown to comprise a user input interface 184 configured to receive user input data 82 from a user input device 80 operable by the user. The user input device 80 may take various forms, including but not limited to a computer mouse, touch screen, keyboard, microphone, etc. Fig. 1 shows the user input device to be a computer mouse 80. In general, the user input interface 184 may be of a type which corresponds to the type of user input device 80, i.e., it may be a thereto corresponding type of user device interface. The user interface subsystem 180 is further shown to comprise a display output interface 182 configured to provide display data 62 to a display 60 to visualize output of the detection system 100. In the example of Fig. 1, the display is an external display 60. Alternatively, the display may be an internal display.

In some examples, the detection system 100 may comprise the aforementioned display output interface but not comprise a user input interface. In some examples, the detection system 100 may comprise another type of output interface, such as an audio interface, e.g., to a loudspeaker, or a network interface. Any of such output interfaces may be used to render or transmit output generated by the detection system 100.

The processor subsystem 140 may be configured to, during operation of the detection system 100, segment the 3D image data 50 into at least two glandular regions, and for each of the glandular regions, provide a separate machine learning model which is trained to detect a region of interest in the 3D image data of a respective glandular region. For example, the at least two separate machine learning models may be accessed as model data 40 from the data storage 20. The processor subsystem 140 may be further configured to, during operation of the detection system 100, apply the separate machine learning model to the 3D image data of the respective glandular region to obtain a detection result, thereby obtaining a plurality of detection results for the glandular regions, and via an output interface, such as the display output interface 182, output detection data indicative of the plurality of detection results. These and other operations of the detection system 100, and various optional aspects thereof, will be explained in more detail elsewhere in this specification.

In general, the detection system 100 may be embodied as, or in, a single device or apparatus. The device or apparatus may be a general-purpose device or apparatus, such as a workstation or a computer, but may also be an application-specific device or apparatus, such as a radiology display workstation. The device or apparatus may comprise one or more microprocessors which may represent the processor subsystem, and which may which execute appropriate software. The software may have been downloaded and/or stored in a corresponding memory, e.g., a volatile memory such as RAM or a non-volatile memory such as Flash. Alternatively, the functional units of the system, e.g., the input interface, the output interface, and the processor subsystem, may be implemented in the device or apparatus in the form of programmable logic, e.g., as a Field-Programmable Gate Array (FPGA). In general, each functional unit of the detection system 100 may be implemented in the form of a circuit. It is noted that the detection system 100 may also be implemented in a distributed manner, e.g., involving different devices or apparatuses. For example, the distribution may be in accordance with a client-server model, e.g., using a server and workstation. For example, the user input interface and the display output interface may be part of the workstation, while the processor subsystem may be a subsystem of the server. It is noted that various other distributions are equally conceivable.

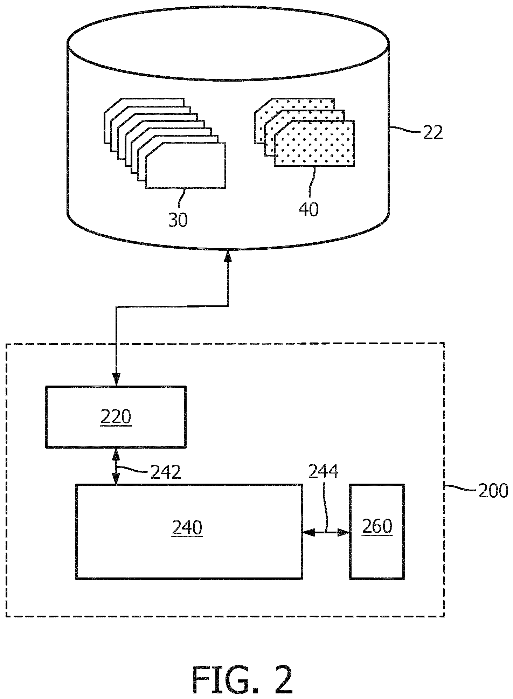

Fig. 2 shows a training system 200 which is configured to train a plurality of machine learning models to detect a region of interest in 3D image data of a prostate of a patient. The plurality of trained machine learning models may then be used by the detection system 100 of Fig. 1 to detect regions of interest in new 3D image data, e.g., of a new patient. The training system 200 is shown to comprise an input interface to access training data 30 to train the machine learning model. In the example of Fig. 2, the input interface is shown to be a data storage interface 220 to an external data storage 22 which comprises the training data 30. The data storage 22 and the data storage interface 220 may for example be of a type as described with reference to Fig. 1 and thereby correspond in type to the data storage 20 and the data storage interface 120 of the detection system 100. However, this is not a limitation, in that the input interface 220 of the training system 200 may also be of any other type, such as a network interface to a Local Area Network (LAN).

The training data 30 may comprise a set of training data instances. Each or at least a subset of the training data instance may comprise 3D image data of a prostate of a patient and annotation data which provides an annotation of one or more regions of interest in the 3D image data. In other words, the 3D image data of a prostate and the accompanying annotation(s) may together form a training data instance, and several training data instances, e.g., from different patients, may together form the training data 30.

The training system 200 may further comprise a processor subsystem 240 configured to internally communicate with the input interface 220 via data communication 242 and with a memory 260 via data communication 244. The memory 260 may for example be a volatile memory in which a computer program may be loaded which may cause the processor subsystem 240 to carry out functions which are described in this specification as being performed by the training system. The processor subsystem 240 may be configured to, during operation of the training system 200, provide a plurality of machine learning models, wherein a separate machine learning model is provided for each of at least two glandular regions of the prostate, and for each or at least a subset of the training data instances, segment the 3D image data into the at least two glandular regions, determine which of the at least two glandular regions comprises a region of interest for which an annotation is provided in the annotation data, and based on said determined glandular region, select and train a corresponding machine learning model on the 3D image data using the annotation of the region of interest as prediction target, thereby obtaining a plurality of trained machine learning models. As a result, a plurality of trained machine learning models may be obtained, which may be stored as model data 40, e.g., in the database 22 and which may be transmitted or in any other way distributed, e.g., to the aforementioned detection system 100 of Fig. 1. These and other operations of the training system 200, and various optional aspects thereof, will be explained in more detail elsewhere in this specification.

In general, the training system 200 may be embodied as, or in, a single device or apparatus, but also in a distributed manner, e.g., involving different devices or apparatuses, and in a same manner as previously described for the detection system 100.

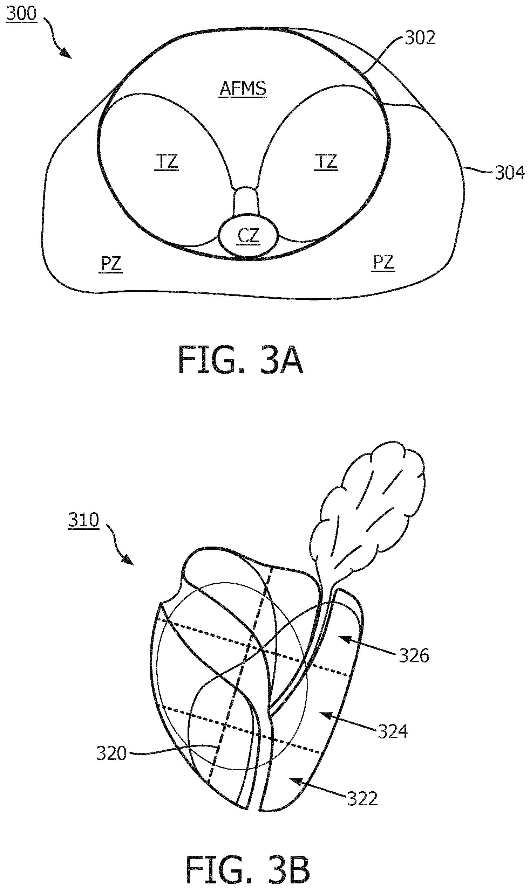

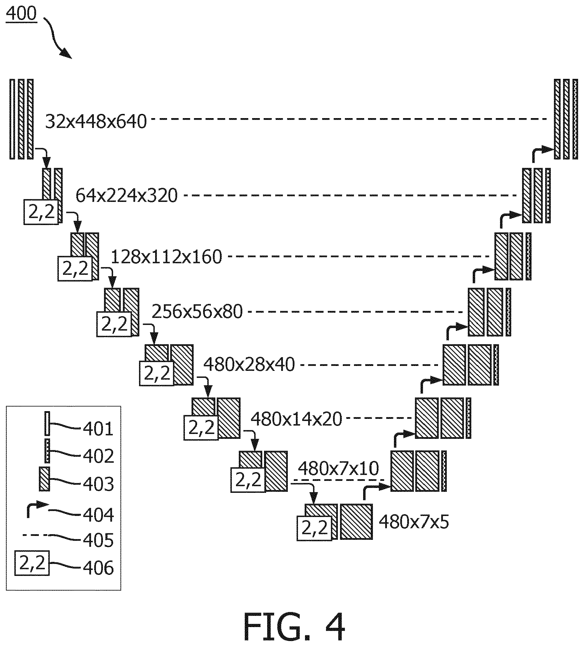

Fig. 3A shows a transverse cross-section 300 of prostate gland, e.g., at 90 degrees to the cranio-caudal axis of the prostate. It can be seen that the prostate gland comprises a plurality of zones, which zones include, but are not limited to, a peripheral zone PZ, a transition zone TZ, a central zone CZ, and an anterior fibromuscular stroma AFMS. The detection system 100 of Fig. 1 and corresponding method may partition the 3D image data of the prostate gland into at least two glandular regions. These glandular regions may later be separately processed by the system and method so as to detect regions of interests in the respective regions. In the example of Fig. 3A, the segmented regions may comprise a peripheral region 304, which may directly correspond to or comprise the peripheral zone PZ, and a central gland region 302. The central gland region 302 may for example comprise the central zone CZ, the transition zone TZ, and the anterior fibromuscular stroma AFMS. Although not explicitly shown in Fig. 3A, the segmentation may be a 3D segmentation, in that it may yield a 3D delineation of the peripheral region 304 and the central gland region 302, for example in form of respective 3D contours or respective 3D masks. The segmentation of the 3D image data into the peripheral region 304 and the central gland region 302 may be provided by a machine learning model which is trained for this purpose. An example of such a machine learning model is described with reference to Fig. 4.

Fig. 3B shows a sagittal cross-section 310 of the prostate gland, e.g., through the cranio-caudal axis 320 of the prostate, and illustrates that in some embodiments, the glandular regions may be divided along the cranio-caudal axis 320 of the prostrate into at least two subregions. In the specific example of Fig. 3B, the subdivision of the glandular regions is shown to be into three subregions, namely an apex region 322 near an apex of the prostate, a base region 326 near a base of the prostate, and a middle region 324 in between the apex region 322 and the base region 326. However, the subdivision along the cranio-caudal axis 320 may also be into two subregions or more than three subregions. In this respect, it is noted that Fig. 3B does not explicitly show the glandular regions as being sub-partitioned but rather illustrates the boundaries of the aforementioned subregions. When partitioning each of the central gland region and the peripheral region into three subregions, six subregions may be obtained, namely a central gland apex region, a central gland middle region, a central gland base region, a peripheral apex region, a peripheral middle region, and a peripheral base region. It will be appreciated, however, that this partitioning is merely exemplary and any other partitioning into glandular regions and sub-partitioning of such glandular regions along the cranio-caudal axis 320 may be used instead.

Fig. 4 shows an architecture 400 of a nnU-Net machine learning model for segmenting glandular regions in 3D image data of a prostate. Here, the term `nnU-Net' refers to the self-adapting framework for U-Net-based medical image segmentation as described by
https://arxiv.org/abs/1809.10486. As is known per se, a nnU-Net machine learning model comprises an encoder, a bottleneck, and a decoder. The encoder, which is also referred to as the `contracting path', is typically composed of blocks, each containing convolutional layers followed by a ReLU activation function and a max-pooling layer for downsampling. This encoder may be used to capture the context of the 3D image data by reducing spatial dimensions while increasing the number of feature channels. The bottleneck, positioned between the encoder and decoder, may comprise convolutional layers to extract dense feature representations. Finally, the decoder may contain blocks with upsampling steps followed by convolutional layers and may restore the spatial dimensions of the feature maps. Skip connections from the encoder may concatenate feature maps to the decoder, helping in reconstruction. Finally, an output layer may provide 1x1 convolution followed by a softmax or sigmoid activation function to for example make pixel-wise predictions. Fig. 4 shows a specific example of such an architecture which is optimized for segmenting the glandular regions in a prostate and in which plain-strided convolution layers are used for downsampling and transposed convolution layers are used for upsampling. As can be seen in Fig. 4, the corresponding strided and transposed convolutions are connected through skip connections. With further reference to Fig. 4, it is noted that numeral 401 refers to an input, numeral 402 to a 1x1 convolution with softmax operator, numeral 403 to a 3x3 convolution using an improved ReLu (`IReLu') as activation function, numeral 404 to a transposed convolution, numeral 405 to a skip connection, and numeral 406 to stride (n, n).

In a particular instance, the nnU-Net model underwent training using the Stochastic Gradient Descent (SGD) optimizer. Nesterov momentum, a variant of momentum that adds some future knowledge to the weight updates, was employed with a value of 0.99 to enhance the optimizer's performance. The learning rate, which controls the step size in weight updates, was set at 0.01 and was gradually reduced using a polyLR scheduler - a strategy that adjusts the learning rate according to a polynomial decay. The training was carried out over 600 epochs. The objective of this training was to minimize a hybrid loss function that combines Binary Cross-Entropy Loss and Dice Loss. Binary Cross-Entropy Loss measures the dissimilarity between the true labels and predicted probabilities, making it suitable for binary classification problems. Dice Loss, on the other hand, is specifically tailored for segmentation tasks, as it measures the overlap between the predicted segmentation and the ground truth. By combining these two loss functions, a better performance was obtained for the segmentation of the glandular regions.

In general, the nnU-Net model may be trained on segmentations of glandular regions provided by radiologist or similar types of ground-truth segmentations. In some embodiments, a machine learning model, such as the aforementioned nnU-Net model, may provide a segmentation of glandular regions while the segmentation of a glandular region into subregions may be carried out using heuristics, for example using a simple function which assigns pixels or voxels within a segmented glandular region to a respective subregion based on its coordinate along the cranio-caudal axis. For example, a middle third of the cranio-caudal axis may be assigned to a middle subregion of a respective glandular region, a top-third to an apex subregion of the glandular region, and a bottom-third to a base subregion of the glandular region. In other embodiments, the machine learning model, such as the aforementioned nnU-Net model, may directly provide a segmentation of glandular regions and the corresponding subregions along the cranio-caudal axis.

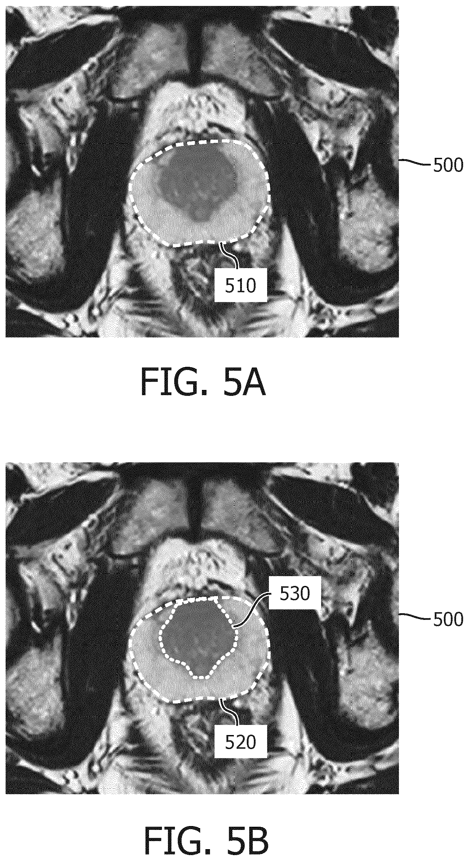

Fig. 5A shows a segmentation 510 of a prostate in an axial image slice 500, which segmentation may be generated by the aforementioned nnU-Net model. As illustrated in Fig. 5B, the nnU-Net model may further segment the prostate into two glandular regions, being in this example the central gland region 530 and the peripheral region 520. The performance of the nnU-Net model was compared to a reference Unet++ model (see https://arxiv.org/pdf/1807.10165) with the respective Dice scores being as follows:

| | | | |
|---|---|---|---|
| | | | |

| Architecture | Prostate | Central gland zone | Peripheral zone |
|---|---|---|---|
| nnU-Net | 0.922 ± 0.07 | 0.858 ± 0.154 | 0.822 ± 0.79 |

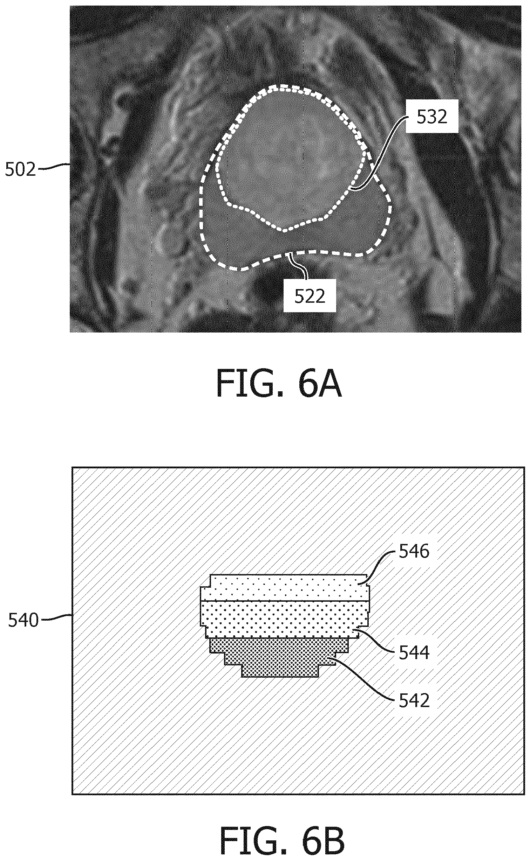

Fig. 6A shows another example of the segmentation of the central gland region 532 and the peripheral region 522 in an axial image slice 502, which segmentation may be generated by the aforementioned nnU-Net model. These glandular regions then be partitioned into subregions. Fig. 6B shows the partitioning into subregions along the cranio-caudal axis, resulting in the partitioning of each of the glandular regions into a respective apex region 542, middle region 544, and base region 546.

Having segmented the 3D image data of the prostate into two or more glandular regions, and optionally a number of subregions along the cranio-caudal axis, separate machine learning models may be applied to each of these (sub)regions. These machine learning models may for example also be nnU-Net models, e.g., having an architecture as described with reference to Fig. 4 and elsewhere in this specification. Each of the machine learning models may be trained to annotate regions of interest in a respective (sub)region. This annotation may for example involve segmenting or highlighting or in any other way annotating the region of interest. In order to train the respective machine learning models, ground truth data may be used, for example in the form of manual annotations of regions of interest obtained from one or more radiologists. In a specific example, the machine learning models may be trained to detect and segment lesions in the 3D image data of a respective (sub)region. At inference time, each of the machine learning models may be applied to the 3D image data of a respective (sub)region. To ensure that a machine learning model is only or predominately applied to the image data of the respective (sub)region, image data belonging to other (sub)regions may be masked-out or cropped-out or the machine learning model may be otherwise selectively applied to the image data of the respective (sub)region. The detection results from each individual machine learning model may then be combined to obtain a combined detection result for entire prostate.

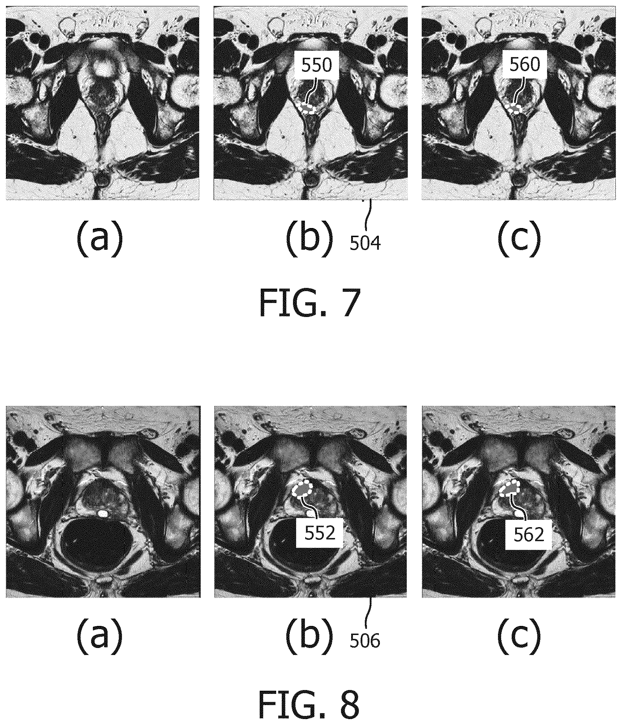

Figs. 7 and 8 each show a comparison between the detection of regions of interest by a machine learning model which is trained to detect regions of interest in the entire prostate region and by a plurality of machine learning models which are specifically trained to each detect regions of interest in different and specific glandular regions. Specifically, in Fig. 7, (a), (b) and (c) are the qualitative outputs of, respectively, a lesion segmentation model which is trained for the whole prostate, a lesion segmentation model which is trained only for the peripheral region, and the ground-truth annotation. In Fig. 8, (a), (b) and (c) are the qualitative outputs of, respectively, a lesion segmentation model which is trained for the whole prostate, a lesion segmentation model which is trained only for the central gland region, and the ground-truth annotation. In both figures, it can be seen that a lesion 550, 552 by the respective specialized model while the whole prostate model is unable to detect the lesion. This is confirmed by the recall rate (sensitivity, true positive rate) as indicated below, from which it follows that the use of multiple specialized segmentation models improves upon the use of one segmentation model for the whole prostate.

| Merged recall from individual specialized models | Recall for whole prostate model |
|---|---|
| 0.623 | 0.6 |

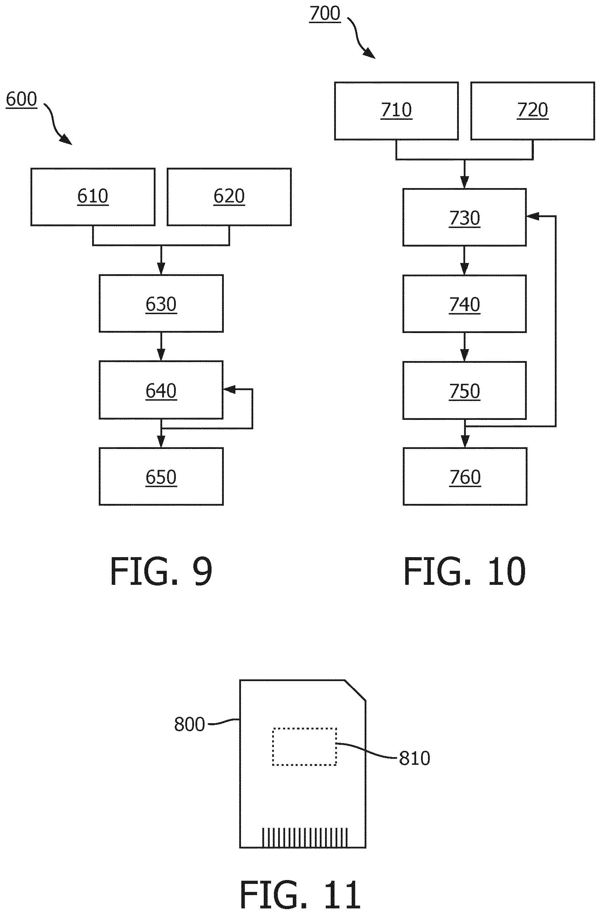

Fig. 9 shows a method 600 for detecting a region of interest in 3D image data of a prostate of a patient using a plurality of machine learning models. The method 600 may correspond to an operation of the detection system 100 of Fig. 1. However, this is not a limitation, in that the computer-implemented method 600 may also be performed using another system, apparatus or device. The method 600 may comprise, in a step titled "ACCESSING 3D IMAGE DATA", accessing 610 the 3D image data, in a step titled "PROVIDING PLURALITY OF TRAINED MACHINE LEARNING MODELS", providing 620 a plurality of trained machine learning models, wherein a separate trained machine learning model is provided for each of at least two glandular regions of the prostate, in a step titled "SEGMENTING 3D IMAGE DATA INTO GLANDULAR REGIONS", segmenting 630 the 3D image data into at least two glandular regions, in a step titled "APPLYING MACHINE LEARNING MODEL TO GLANDULAR REGION", for each of the glandular regions, applying 640 the separate machine learning model to the 3D image data of the respective glandular region to obtain a detection result, and in a step titled "OUTPUTTING DETECTION RESULTS", outputting 650 detection data indicative of the plurality of detection results.

Fig. 10 shows a method 700 for training a plurality of machine learning models to detect a region of interest in 3D image data of a prostate of a patient. The method 700 may correspond to an operation of the training system 200 of Fig. 2. However, this is not a limitation, in that the computer-implemented method 700 may also be performed using another system, apparatus or device. The method 700 may comprise, in a step titled "ACCESSING TRAINING DATA", accessing 710 training data as described elsewhere in this specification, in a step titled "PROVIDING PLURALITY OF MACHINE LEARNING MODELS", providing 720 a plurality of machine learning models as described elsewhere in this specification, in a step titled "SEGMENTING 3D IMAGE DATA INTO GLANDULAR REGIONS", segmenting 730 the 3D image data into the at least two glandular regions, in a step titled "DETERMINING GLANDULAR REGION", determining 740 which of the at least two glandular regions comprises a region of interest for which an annotation is provided in the annotation data, in a step titled "TRAINING MACHINE LEARNING MODEL ON GLANDULAR REGION", based on said determined glandular region, selecting and training 750 a corresponding machine learning model on the 3D image data using the annotation of the region of interest as prediction target, and in a step titled "OUTPUTTING MODEL DATA", outputting 760 model data defining the plurality of trained machine learning models.

It will be appreciated that steps of the method 600 of Fig. 9 and the method 700 of Fig. 10 may be performed in any suitable order, e.g., consecutively, simultaneously, or a combination thereof, subject to, where applicable, a particular order being necessitated, e.g., by input/output relations. Steps may also be performed as part of other steps.

Any method described in this specification may be implemented on a computer as a computer-implemented method, as hardware, or as a combination of both. As also illustrated in Fig. 11, instructions for the computer, e.g., executable code, may be stored on a computer readable medium 800, e.g., in the form of a series 810 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, etc. Fig. 8 shows a memory device 800.

Examples, embodiments or optional features, whether indicated as non-limiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

### List of reference numbers

The following list of reference numbers is provided for facilitating the interpretation of the drawings and shall not be construed as limiting the claims.
- AFMS: anterior fibromuscular stroma
- CZ: central zone
- PZ: peripheral zone
- TZ: transition zone

- 20, 22: data storage
- 30: training data
- 40: machine learning models
- 50: 3D image data of prostate
- 60: display
- 62: display data
- 80: user input device
- 82: user input data

- 100: detection system
- 110: data storage interface
- 120: input interface
- 140: processor subsystem
- 142-148: data communication
- 160: memory
- 180: user interface subsystem
- 182: display output interface
- 184: user input interface

- 200: training system
- 220: data storage interface
- 240: processor subsystem
- 242-246: data communication
- 260: memory

- 300: transverse cross-section of prostate gland
- 302: central gland region
- 304: peripheral region
- 310: sagittal cross-section of prostate gland
- 320: cranio-caudal axis
- 322: apex
- 324: middle
- 326: base

- 400: nnU-Net architecture
- 401: input
- 402: 1x1 convolution - softmax
- 403: 3x3 convolution - IreLu
- 404: transposed convolution
- 405: skip connection
- 406: stride (n, n)

- 500-506: axial image slice
- 510: segmentation of prostate
- 520, 522: segmentation of peripheral region
- 530, 532: segmentation of central gland region
- 540: cranio-caudal axis view
- 542: apex region
- 544: middle region
- 546: base region
- 550, 552: detected region of interest
- 560, 562: ground truth region of interest

- 600: method of detecting region of interest in image data of prostate
- 610: accessing 3D image data
- 620: providing plurality of trained machine learning models
- 630: segmenting 3D image data into glandular regions
- 640: applying machine learning model to glandular region
- 650: outputting detection results

- 700: method of training machine learning models to detect region of interest in image data prostate
- 710: accessing training data
- 720: providing plurality of machine learning models
- 730: segmenting 3D image data into glandular regions
- 740: determining glandular region
- 750: training machine learning model on glandular region
- 760: outputting model data

- 800: non-transitory computer-readable medium
- 810: data representing computer program

## Claims

1. A computer-implemented method (600) for detecting a region of interest in three-dimensional [3D] image data of a prostate of a patient, comprising:
- accessing (610) the 3D image data (50);
- segmenting (630) the 3D image data into at least two glandular regions (302, 304);
- for each of the glandular regions:
- providing (620) a separate machine learning model (40) which is trained to detect a region of interest in the 3D image data of a respective glandular region,
- applying (640) the separate machine learning model to the 3D image data of the respective glandular region to obtain a detection result (550, 552),
thereby obtaining a plurality of detection results for the glandular regions; and
- outputting (650) detection data indicative of the plurality of detection results.

2. The method (600) according to claim 1, wherein the at least two glandular regions include a peripheral region (304) of the prostrate and a central gland region (302) of the prostrate.

3. The method (600) according to claim 1 or 2, wherein the central gland region (302) comprises an anterior fibromuscular stroma (AFMS), a transition zone (TZ), and a central zone (CZ) of the prostate.

4. The method (600) according to any one of claims 1 to 3, further comprising segmenting each of the at least two glandular regions into at least two subregions (322-326, 542-546) which divide a respective glandular region along a cranio-caudal axis (320) of the prostrate, wherein a separate machine learning model is applied to the image data of each of the at least two subregions.

5. The method (600) according to claim 4, wherein the at least two subregions (322-326, 542-546) comprise a base subregion (326, 546) at a base of the prostate and an apex subregion (322, 542) at an apex of the prostrate.

6. The method (600) according to claim 5, further comprising segmenting each of the at least two glandular regions along the cranio-caudal axis into at least three subregions (322-326, 542-546), wherein the at least three subregions comprise the base subregion (326, 546), the apex subregion (322, 542), and a middle subregion (324, 544) positioned between the base subregion and the apex subregion along the cranio-caudal axis.

7. The method (600) according to any one of claims 4 to 6, wherein the segmentation into glandular regions (302, 304) is performed using a machine learning model and wherein the segmentation into subregions is performed using heuristics.

8. The method (600) according to any one of claims 1 to 7, wherein applying the separate machine learning model to the 3D image data of the respective glandular region (302, 304) comprises masking-out or cropping-away image data outside of the respective glandular region.

9. The method (600) according to any one of claims 1 to 8, wherein the 3D image data (50) is obtained by magnetic resonance imaging, for example using a T2 sequence.

10. The method (600) according to any one of claims 1 to 9, wherein a respective machine learning model is a neural network comprising one or more convolutional layers.

11. A computer-implemented method (700) for training a plurality of machine learning models to detect a region of interest in three-dimensional [3D] image data of a prostate of a patient, comprising:
- accessing (710) training data (30) comprising a plurality of training data instances, wherein a training data instance comprises 3D image data of a prostate of a patient and annotation data which provides an annotation of one or more regions of interest in the 3D image data;
- providing (720) a plurality of machine learning models (40), wherein a separate machine learning model is provided for each of at least two glandular regions of the prostate;
- for each or at least a subset of the training data instances:
- segmenting (730) the 3D image data into the at least two glandular regions (302, 304),
- determining (740) which of the at least two glandular regions comprises a region of interest for which an annotation is provided in the annotation data, and
- based on said determined glandular region, selecting and training (750) a corresponding machine learning model on the 3D image data using the annotation of the region of interest as prediction target,
thereby obtaining a plurality of trained machine learning models;
- outputting (760) model data defining the plurality of trained machine learning models.

12. A transitory or non-transitory computer-readable medium (800) comprising data (810) representing a computer program, the computer program comprising instructions for causing a processor system to perform the method according to any one of claims 1 to 11.

13. A detection system (100) for detecting a region of interest in three-dimensional [3D] image data of a prostate of a patient, comprising:
- an input interface (120) for accessing the 3D image data (50);
- a processor subsystem (140) configured to:
- segment the 3D image data into at least two glandular regions;
- for each of the glandular regions:
- provide a separate machine learning model (40) which is trained to detect a region of interest in the 3D image data of a respective glandular region,
- apply the separate machine learning model to the 3D image data of the respective glandular region to obtain a detection result,
thereby obtaining a plurality of detection results for the glandular regions; and
- an output interface (182) for outputting detection data indicative of the plurality of detection results.

14. The detection system (100) according to claim 13, wherein the output interface is a display output interface (182) for visualizing the plurality of detection results on a display (60), for example as an overlay over the 3D image data.

15. A training system (200) for training a plurality of machine learning models to detect a region of interest in three-dimensional [3D] image data of a prostate of a patient, comprising:
- an input interface (220) for accessing training data (30) comprising a plurality of training data instances, wherein a training data instance comprises 3D image data of a prostate of a patient and annotation data which provides an annotation of one or more regions of interest in the 3D image data;
- a processor subsystem (240) configured to:
- provide a plurality of machine learning models (40), wherein a separate machine learning model is provided for each of at least two glandular regions of the prostate
- for each or at least a subset of the training data instances:
- segment the 3D image data into the at least two glandular regions,
- determine which of the at least two glandular regions comprises a region of interest for which an annotation is provided in the annotation data, and
- based on said determined glandular region, select and train a corresponding machine learning model on the 3D image data using the annotation of the region of interest as prediction target, thereby obtaining a plurality of trained machine learning models; and
- an output interface (220) for outputting model data defining the plurality of trained machine learning models.
